# EUROPEAN PATENT APPLICATION

(11) **EP 4 697 348 A1**
(43) Date of publication of application: **18.02.2026**
(21) Application number: 24194608.6
(22) Date of filing: 14.08.2024
(51) Int. Cl.: G16H 30/40

(54) **RECOGNIZING PERSONAL DATA IN MEDICAL IMAGES**

(71) Applicant: Bayer Aktiengesellschaft, 51373 Leverkusen (DE)
(72) Inventor: LENGA, Matthias, 51373 Leverkusen (DE); BALTRUSCHAT, Ivo Matteo, 51373 Leverkusen (DE); TRUONG, Dihn Tuan, 51373 Leverkusen (DE); BOSE, Subrata, RG2 6AD Reading (GB)
(74) Representative: BIP Patents

(57) **Abstract**

Systems, methods, and computer programs disclosed herein relate to the recognition of personal data in medical images.

## Description

### FIELD OF THE DISCLOSURE

Systems, methods, and computer programs disclosed herein relate to the recognition of personal data in medical images.

### BACKGROUND

Medical images, including but not limited to X-rays, magnetic resonance imaging (MRI) images, computed tomography (CT) scans, positron emission tomography (PET) scans, and ultrasound images, serve as a cornerstone for clinical decision-making. These images capture detailed internal structures and functions of the body, providing clinicians with invaluable data for diagnosing a wide array of conditions, from fractures and tumours to infectious diseases and congenital anomalies. The depth and breadth of information that can be gleaned from medical images are vast, encompassing anatomical details, physiological functions, and even molecular processes.

Medical images can be automatically analysed using artificial intelligence methods to segment the images, identify tissue types, detect signs of disease, track the progression of a disease and/or the treatment of a disease, and much more.

Medical images can be used to train machine learning models that automatically process and analyse medical images.

Medical images are thus a valuable resource.

However, medical images are also sensitive data.

Medical images provide detailed insights into a person's health. They can reveal diagnoses, medical conditions, and the results of medical procedures. This information is highly personal and can be misused if it falls into the wrong hands.

Medical images often contain identifiable information about patients. Even if the images themselves don't directly reveal a person's identity, they are usually linked to other personal data like names, dates of birth, and medical record numbers.

Unauthorized access to medical images can lead to misuse, including identity theft, insurance fraud, and discrimination.

It is therefore important to know whether medical images contain personal data.

### SUMMARY

The present disclosure addresses this and further needs.

In a first aspect, the present disclosure provides a computer-implemented method comprising:
- receiving patient data, wherein the patient data comprises a medical image,
- recognizing text in the medical image,
- recognizing personal data in the text,
- issuing a notification about the personal data and/or deletion of the personal data.

In another aspect, the present disclosure provides a computer system comprising:
a processing unit; and
a memory storing a computer program configured to perform, when executed by the processing unit, an operation, the operation comprising:
   - receiving patient data, wherein the patient data comprises a medical image,
   - recognizing text in the medical image,
   - recognizing personal data in the text,
   - issuing a notification about the personal data and/or deletion of the personal data.

In another aspect, the present disclosure provides a non-transitory computer readable storage medium having stored thereon a computer program that, when executed by a processing unit of a computer system, cause the computer system to execute the following steps:
- receiving patient data, wherein the patient data comprises a medical image,
- recognizing text in the medical image,
- recognizing personal data in the text,
- issuing a notification about the personal data and/or deletion of the personal data.

### BRIEF DESCRIPTION OF THE DRAWINGS

Fig. 1 shows a schematic and exemplary embodiment of the computer-implemented method of the present disclosure.
Fig. 2 shows an exemplary and schematic architecture underlying the computer-implemented method, computer program and computer system of the present disclosure.
Fig. 3 shows a variant of the architecture shown in Fig. 2.
Fig. 4 shows a variant of the architectures shown in Fig. 2 and Fig. 3.
Fig. 5, Fig. 6 and Fig. 7 schematically and exemplary show the removal of personal data from medical images.
Fig. 8 shows an embodiment of the computer-implemented method of the present disclosure in the form of a flow chart.
Fig. 9 shows another embodiment of the computer-implemented method of the present disclosure in the form of a flow chart.
Fig. 10 illustrates a computer system according to some example implementations of the present disclosure in more detail.

### DETAILED DESCRIPTION

Various example embodiments will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one". As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides means for recognizing personal data in medical images.

"Personal data", also known as personally identifiable information (PII), refers to any information that relates to an identified or identifiable individual. This includes, but is not limited to, data that can directly or indirectly identify a person.

An "identifiable person" is one who can be identified, directly or indirectly, in particular by reference to an identifier such as a name, an identification number, location data, an online identifier, or to one or more factors specific to the physical, physiological, genetic, mental, economic, cultural, or social identity of that natural person.

Examples of personal data include name, postal address, e-mail address, phone number, social security number, passport number, insurance number, patient ID, IP address, device identifier, geolocation data, date of birth, weight, height, and others.

In the European Union (EU), personal data is defined under the General Data Protection Regulation (GDPR), which came into effect on May 25, 2018. According to Article 4(1) of the GDPR, personal data means any information relating to an identified or identifiable natural person.

In an embodiment of the present disclosure, the personal data includes protected health information (PHI), also referred to as personal health information.

Protected health information (PHI) refers to any information in a medical record or other health-related information that can be used to identify an individual and that was created, used, or disclosed in the course of providing a healthcare service such as diagnosis or treatment. This information can include a wide range of identifiers beyond just medical records, extending to demographic information, test and laboratory results, insurance information, and any other data that a healthcare professional collects to identify an individual and to provide healthcare services.

Examples of protected health information include but are not limited to name and contact information (this includes full names, addresses (e.g., postal and/or e-mail), phone numbers, and any other contact information that can directly identify an individual), medical records (e.g., records of medical history, diagnoses, treatments, and/or prescriptions), insurance information (e.g. details regarding an individual's health insurance policy, including the policy number and/or insurance company), birth date, admission date, discharge date (e.g., an individual's admission, and discharge from a healthcare facility), social security number, and medical device identifiers (e.g., information related to any medical devices that are attached or implanted in patients).

For example, the Health Insurance Portability and Accountability Act (HIPAA) in the United States sets standard for protecting sensitive patient data, including PHI.

In a first step, patient data is received.

The term "receiving" is to be interpreted broadly and includes any means capable of providing patient data for further processing by the computer system of the present disclosure.

The term "receiving" may mean transmitting data from another computer system to the computer system of the present disclosure. The term "receiving" may mean transmitting data from a medical imaging device such as a CT scanner, an MRI scanner, an ultrasound scanner, a PET scanner, an OCT scanner, a microscope, and/or the like to the computer system of the present disclosure. The term "receiving" may mean entering of data into the computer system of the present disclosure, e.g. by a user. The term "receiving" may mean retrieving data from a data storage; the data storage may be a component of and/or connected to the computer system of the present disclosure, for example via a network.

"Patient data" is data relating to a living being, usually a human being.

The patient data comprises one or more medical image(s).

The term "image" as used herein means a data structure that represents a spatial distribution of a physical signal. The spatial distribution may be of any dimension, for example 2D, 3D, 4D or any higher dimension. The spatial distribution may be of any shape, for example forming a grid and thereby defining pixels or voxels, the grid being possibly irregular or regular. The physical signal may be any signal, for example proton density, tissue echogenicity, tissue radiolucency, measurements related to the blood flow, information of rotating hydrogen nuclei in a magnetic field, color, level of gray, depth, surface or volume occupancy, such that the image may be a 2D or 3D RGB/grayscale/depth image, or a 3D surface/volume occupancy model. An image is usually composed of discrete image elements (e.g., pixels for 2D images, voxels for 3D images, doxels for 4D images).

A "medical image" is a representation of the patient's body or a part thereof. Medical images can be used, e.g., for diagnostic and/or treatment purposes.

Techniques for generating medical images include X-ray radiography, computerized tomography, fluoroscopy, magnetic resonance imaging, ultrasonography, endoscopy, elastography, tactile imaging, thermography, microscopy, positron emission tomography, optical coherence tomography, fundus photography, and others.

Examples of medical images include CT (computer tomography) scans, X-ray images, MRI (magnetic resonance imaging) scans, PET (positron emission tomography) scans, fluorescein angiography images, OCT (optical coherence tomography) scans, microscopic images, histological images, ultrasound images, fundus images and/or others.

In an embodiment of the present disclosure, the medical image is a radiologic image. "Radiology" is the branch of medicine concerned with the application of electromagnetic radiation and mechanical waves (including, for example, ultrasound diagnostics) for diagnostic, therapeutic and/or scientific purposes.

In addition to X-rays, other ionizing radiation such as gamma rays or electrons are also used. Since a primary purpose is imaging, other imaging procedures such as sonography and magnetic resonance imaging (MRI) are also included in radiology, although no ionizing radiation is used in these procedures. Thus, the term "radiology" as used in the present disclosure includes, in particular, the following examination procedures: computed tomography, magnetic resonance imaging, sonography, positron emission tomography.

The medical image may be a representation of an examination region of the patient generated with or without a contrast agent.

"Contrast agents" are substances or mixtures of substances that improve the depiction of structures and functions of the body in radiological examinations. In computed tomography, iodine-containing solutions are usually used as contrast agents. In magnetic resonance imaging (MRI), superparamagnetic substances (for example iron oxide nanoparticles, superparamagnetic iron-platinum particles (SIPPs)) or paramagnetic substances (for example gadolinium chelates, manganese chelates, hafnium chelates) are usually used as contrast agents. In the case of sonography, liquids containing gas-filled microbubbles are usually administered intravenously. In positron emission tomography (PET) radiotracers are used as contrast agents.

In an embodiment of the present disclosure, the medical image is an MRI image.

In another embodiment of the present disclosure, the medical image is a CT image.

In an embodiment of the present disclosure, the medical image is a PET image.

In an embodiment of the present disclosure, the medical image is an X-Ray image.

In an embodiment of the present disclosure, the medical image is a bone scan image.

In an embodiment of the present disclosure, the medical image is an ultrasound image.

In an embodiment of the present disclosure, the medical image is a microscopic image of a tissue sample.

The medical image represents an examination region of the patient.

The "examination region" is a part of the patient, for example an organ or part of an organ or a plurality of organs or another part of the patient. The examination region can also be part of a sample, e.g. part of a tissue sample.

For example, the examination region may be a liver, kidney, heart, lung, brain, stomach, bladder, prostate, intestine, thyroid, eye, breast, skin, pancreas, or a part of said parts or another part of the body of a mammal (for example a human).

The medical image is analysed to identify text in the image.

Recognizing text in the medical image may comprise detecting one or more text regions in the medical image.

"Text region detection" is the process of identifying areas within the medical image that contain text. Text region detection focuses on locating and marking where text exists within the broader context of an image.

Text region detection can be approached through several methods, ranging from traditional computer vision techniques to advanced deep learning models.

In case of traditional computer vision techniques, techniques like the Canny edge detector can highlight edges in the medical image, which may then be analysed to identify patterns indicative of text. Morphological operations such as erosion, dilation, opening, and closing operations can help emphasize text structures or remove non-text elements, making it easier to identify text regions. Connected components analysis can be used to group adjacent pixels into clusters, which may represent characters or blocks of text.

Traditional machine learning approaches for text region detection involve extracting hand-crafted features (such as histogram of oriented gradients (HOG) features) from images and using classifiers (e.g., support vector machine, random forests) to distinguish text from non-text regions.

Convolutional neural networks (CNN) can be trained on large datasets of annotated images to learn features that are indicative of text, eliminating the need for manual feature engineering.

Region-based convolutional neural networks (R-CNN) and its variants like Faster R-CNN (arXiv:1506.01497v3), YOLO (You Only Look Once, arXiv:1506.02640v5), and SSD (Single Shot MultiBox Detector, arXiv: 1512.02325v5) can efficiently detect text regions in an image. They can predict bounding boxes and class labels for text regions in a single forward pass, making them efficient and scalable for real-world applications.

Fully convolutional networks (FCNs, arXiv:2005 . 14229v 1) can perform pixel-level classification, determining for each pixel whether it belongs to a text region or not.

Recognizing text in the medical image may comprise text region extraction.

"Text region extraction" refers to the process of isolating regions within the medical image that contain textual content. This step narrows down the areas of the medical image that need to be analysed for text, thereby increasing the efficiency and accuracy of the recognition process.

Text region extraction may comprise segmenting text regions from the rest of the medical image.

Text region extraction may comprise segmenting text into lines, words, and/or individual characters.

Recognizing text in the medical image may comprise perspective correction and/or normalization.

Text in medical images can be skewed, rotated, or distorted, e.g. due to the angle of the camera or the positioning of the imaging device.

"Perspective correction" refers to the process of transforming an image (e.g. the image of the extracted text region) to correct such distortions. Perspective correction ensures that text appears as if it were viewed head-on, making it easier to read and recognize by both humans and automated systems.

"Normalization" refers to a series of preprocessing steps aimed at transforming the text in images (e.g. the image of the extracted text region) into a more uniform and standard format, making it easier for optical character recognition (OCR) algorithms and/or machine learning models to process and recognize the text accurately.

Normalization may include size normalization, i.e. adjusting the size of the text or the entire image so that all text elements are of a consistent size.

Normalization may include intensity normalization, i.e. adjusting the brightness and contrast of the image to make the text more distinguishable from the background.

Normalization may include grayscale conversion, i.e. converting colour images to grayscale to reduce computational complexity and focus the algorithm on text shape and structure rather than colour differences.

Normalization may include binarization, i.e. converting grayscale images to binary (black and white) images, where the text is typically represented in black and the background in white.

Normalization may include slant correction, i.e. adjusting the slant of cursive or italicized text to resemble upright (vertical) text, which is typically easier for OCR systems to recognize.

Normalization may include noise reduction, i.e. removing artifacts and noise from the image that could interfere with text recognition. Noise reduction may involve filtering techniques that smooth out the image without blurring the text.

Recognizing text in the medical image may comprise transcribing visual information of a text region into textual data.

"Transcribing visual information of a text region into textual data" refers to the process of converting the graphical representation of text (as seen in the image) into machine-encoded text (such as ASCII or Unicode characters) that can be easily processed, stored, and understood by computers and software applications.

Transcribing visual information of a text region into textual data may include character recognition. Each character within the text region may be recognized and converted into a corresponding textual representation. This can be accomplished, e.g. using template matching, i.e. comparing each character against a predefined set of character templates to find the best match.

Machine learning models, e.g. deep learning approaches like CNNs combined with recurrent neural networks (RNNs) and long short-term memory (LSTM) networks can also be used to recognize characters. These models can learn complex patterns in the visual appearance of text and are capable of handling a wide variety of fonts, styles, and distortions (see, e.g., G. Sarker et al.: A Convolution Neural Network for Optical Character Recognition and Subsequent Machine Translation, International Journal of Computer Applications (0975 - 8887) Volume 182 - No. 30, December 2018).

In case characters are not segmented before recognition, connectionist temporal classification (CTC) can be used for aligning input sequences (images of text lines or words) with their corresponding textual labels in an end-to-end manner (see, e.g., arXiv:2402.05417v1).

Recognizing text in the medical image may comprise post-processing steps such as spell checking, and context-based corrections to improve accuracy.

Recognizing text in the medical image may comprise completing, cleaning and/or structuring the text in a meaningful way (e.g. considering standard medical terms).

In an embodiment of the present disclosure, a pre-trained language model is used to correct the text and/or add missing letters and/or words and/or characters and/or complete, clean and/or structure the text in a meaningful way.

A "language model", also referred to as "large language model" (LLM) is a type of artificial intelligence (AI) system that utilizes deep learning techniques, particularly neural networks with a vast number of parameters, to understand, generate, and manipulate human language. These models are trained on extensive datasets comprising vast amounts of text from diverse sources to learn the statistical properties and structures of language. The primary objective of a language model is to perform a wide range of natural language processing (NLP) tasks, such as text generation, translation, summarization, question answering, and sentiment analysis, with high accuracy and contextual understanding. Language models may be based on transformer networks.

The term "pre-trained" refers to a machine learning model that has undergone an initial training phase on a large and diverse dataset before being fine-tuned and/or applied to specific tasks. Pre-training involves learning the underlying patterns, structures, and representations of data, which can then be leveraged for various downstream tasks with minimal additional training. Pre-trained models provide a strong starting point, significantly reducing the computational resources and time required to develop effective AI systems for specific applications.

"Fine-tuning" refers to the process of taking a pre-trained machine learning model and further training it on a task-specific dataset to adapt it to a particular application or domain. Fine-tuning leverages the general knowledge that the machine learning model has already acquired during its initial pre-training phase and adjusts it to perform optimally on a specific task.

The text recognized during text recognition may be fed to a language model, and the language model may be configured to make linguistic and/or grammatical corrections, correct spelling errors, add missing letters, characters and/or words, and output a corrected text. The language model may be pre-trained. The language model may be trained/fine-tuned based on text commonly found in medical records and/or medical images.

In a further step, the (optionally corrected) text is analysed to identify personal data in the text.

A language model can be used for this recognition of personal data.

The language model can be pre-trained. The language model can be trained and/or fine-tuned based on texts containing personal data. The language model can be trained and/or fine-tuned at least partially based on texts that usually occur in medical records and/or medical images.

The language model can be configured as a classifier. The classifier can be a binary classifier or a multi-classifier.

The binary classifier can be configured to indicate whether a text (e.g. the text in a text region) includes personal data.

The binary classifier can be configured to indicate for individual words or groups of words or dates or digits or groups of digits whether it is personal data (e.g. a name or a date of birth or a weight or the like) or not.

The multi-classifier can be configured to indicate for individual words or groups of words or dates or digits or groups of digits whether it is personal data (e.g. a name or a date of birth or a weight or the like) and what type of personal data it is.

The language model may be configured to specify a probability for the correctness of the classification.

In addition to the text, the language model can be fed with further data that helps the model to recognize personal data.

In addition to the medical image, the patient data received may include information about the patient, such as the patient's name, postal address, e-mail address, phone number, social security number, passport number, insurance number, patient ID, IP address, date of birth, weight, height, and/or others.

Data on the medical image can also be added to the language model, such as the type of medical image, the region of examination, the day the medical image was generated, the device used to generate the medical image, the location where the medical image was generated, the institution from which the medical image was generated and/or other data.

If the language model is given such data, it is easier for the language model to recognize personal data in the text of the medical image.

In the next step, a notification can be issued about the presence of personal data in the medical image.

The notification may indicate whether the medical image contains personal data or not.

The notification may indicate the likelihood that the medical image includes personal data.

The notification may indicate what type(s) (e.g. name, or date of birth or address or the like) of personal data the medical image contains.

For each type, the notification may specify the probability that the medical image contains the type of personal data.

The notification may include the personal data itself.

In an embodiment of the present disclosure, a message about the presence of personal data is only issued if the probability of the presence of the personal data is above a threshold value.

The threshold value can, for example, be 50% or 55% or 60% or 65% or 67% or 70% or another value.

The threshold value may be set by a user of the computer program of the present disclosure.

The notification may indicate where personal data is present in the medical image and/or where personal data is present with a probability above a threshold or where personal data of a defined type is present in the medical image.

The notification may include the medical image in which text representing personal data is marked. Text can be marked by a bounding box and/or colour coded.

The colour and/or type of bounding box can indicate the type of personal data.

The notification may include the recognized text.

The notification may include a list of all texts identified in the medical image and an indication of whether the text is personal data, the likelihood that it is personal data, what type of personal data the text represents, the likelihood that the text represents the type of personal data and/or an explanation of why the text is personal data and/or personal data of a particular type.

In an embodiment of the present disclosure, text representing personal data is automatically removed from the medical image. "Automatically" means without human intervention. The terms "remove" and "delete" are used synonymously in this disclosure; this also applies to the terms "removal" and "deletion".

For example, it is possible that the grey or colour values of the image elements (e.g. pixels) of the medical image within a bounding box containing text with personal data are set to zero or another value.

For example, it is possible that the grey or colour values of the image elements (e.g. pixels) of the medical image that represent text that represents personal data are set to zero or another value.

In an embodiment of the present disclosure, an image-to-image translation model is used to remove text from the medical images.

The image-to-image translation model can be a generative adversarial network (GAN), such as a conditional GAN (cGAN). One popular architecture of cGANs used for image-to-image translation tasks is Pix2Pix. A GAN comprises two main components: a generator and a discriminator. The generator takes a (medical) image with text as input and tries to generate a (medical) image without text. It's typically implemented as a U-Net, which is a type of convolutional neural network (CNN) that excels in image-to-image translation tasks. The discriminator tries to distinguish between the real images (without text) and the generated images (from the generator). It may be implemented as a PatchGAN, which classifies each patch of the image as real or fake and then averages the results.

The image-to-image translation model can be trained on training data comprising (medical) images with text and the corresponding images without text. The image-to-image translation model may be trained in a supervised learning task. It should be noted that the image-to-image translation model does not necessarily have to be trained based on medical images. However, it is advantageous to at least fine-tune a model that has been trained on images other than medical images, based on medical images. The training data can be generated at least partially by adding text to (medical) images without text. Texts that normally appear in medical images can be used.

In training, a combination of two loss functions may be used, such as adversarial loss and L1 loss. Adversarial loss encourages the generator to generate images that the discriminator cannot distinguish from real images. L1 loss encourages the generator to generate images that are close to the target images in the L1 norm sense.

The text that is to be removed from the (medical) image can also be entered into the image-to-image translation model as additional input data when training the model and during inference. The text can be entered into the model as an image (graphical representation) and/or in the form of a text embedding. Such a text embedding can be generated using a language model and/or a multimodal model such as the CLIP model (see, e.g., C. Che *et al.: Enhancing Multimodal Understanding with CLIP-Based Image-to-Text Transformation,* arXiv:2401.06167v1, 2024).

In an embodiment of the present disclosure, a diffusion model is used to remove text from the medical image.

Diffusion models are a type of generative model that may simulate a Markov chain to gradually transform a given data distribution into a known prior distribution (like Gaussian noise). The transformation is achieved through a series of small diffusion steps, hence the name.

For the task of removing text from medical images, a diffusion model may be trained to learn the distribution of clean images (without text) and then used to sample from this distribution given a noisy image (with text). The model would effectively "diffuse" the text away through a series of small transformations.

Similar to the GAN approach, training data comprising (medical) images with and without text may be used.

The diffusion model usually includes a neural network that predicts the parameters of the Gaussian noise to be added at each diffusion step. The diffusion model may be trained to learn the distribution of clean (medical) images. This typically involves a maximum likelihood estimation approach, where the model is trained to maximize the likelihood of the clean images under its learned distribution.

To remove text from a new medical image, a noisy image is used as a starting point and the diffusion process is run in reverse. At each step, the diffusion model predicts the parameters of the Gaussian noise to be subtracted, effectively "denoising" the image.

The aspects of the present disclosure are explained in more detail below with reference to the drawings, without the intention of limiting the aspects of the present disclosure to the embodiments, features and/or combinations of features shown in the drawings. Statements made with reference to individual drawings are not limited to the particular embodiment shown but are also intended to apply (analogously) to other embodiments.

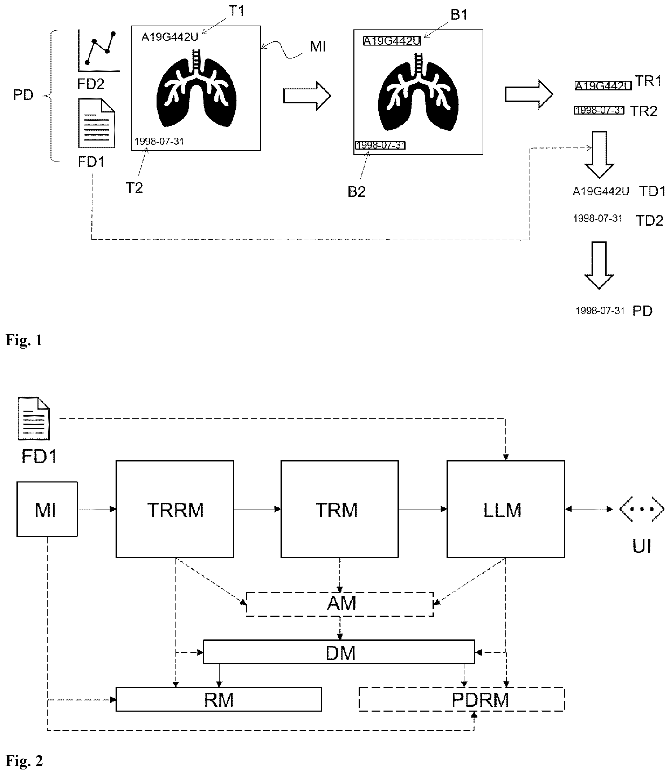

Fig. 1 shows a schematic and exemplary embodiment of the computer-implemented method of the present disclosure.

In a first step patient data PD is received. The patient data PD comprises a medical image MI and additional data FD1, FD2. The medical image MI shows a human lung. The medical image MI includes text T1, T2. The additional data FD1, FD2 may be text data, numbers, one or more further (medical) images and/or other data.

In a further step, regions in the medical image MI that contain text are recognized. The text is marked by bounding boxes B1 and B2.

In a further step, the text regions TR1 and TR2 are extracted.

In a further step, the characters in the text regions are recognized, i.e. the graphical representations of the text are transcribed into text data TD 1 and TD2. Additional data, for example the additional data FD1, can be used for the transcription.

In a further step, personal data PD is recognized in the text data TD 1 and TD2. The personal data PD could be a date of birth.

Fig. 2 shows an exemplary and schematic architecture underlying the computer-implemented method, computer program and computer system of the present disclosure.

The architecture comprises a text region recognition module (TRRM), a text recognition module (TRM), a language model (LM), an aggregation module (AM), a decision module (DM), a reporting module (RM) and a personal data removal module (PDRM). In the example of Fig. 2, the aggregation module (AM), and the personal data removal module (PDRM) are optional (indicated by the dashed lines).

A medical image MI is fed to the text region recognition module TRRM. The text region recognition module TRRM is configured to recognize regions within the medical image MI that contain text. The text region recognition module TRRM may be configured to provide the recognized text region with a bounding box. The text region recognition module TRRM may be configured to provide the coordinates of the bounding box to the aggregation module AM, the decision module DM and/or the reporting module RM. The text region recognition module TRRM may be configured to extract regions with text from the medical image MI. The text region recognition module TRRM may be configured to provide the extracted text regions to the text recognition module TRM, the aggregation module AM, the decision module DM and/or the reporting module RM. The text region recognition module TRRM may be or include a YOLO model (https://pjreddie.com/darknet/yolo/), for example.

The text recognition module TRM is configured to convert the graphical representation of text in the medical image into text data. The text recognition module TRM is configured to provide the text data to the language model LLM. The text recognition module TRM may be or include an optical character recognition module such as Tesseract (https://github.com/tesseract-ocr/tesseract), for example. In another embodiment, the text recognition module TRM may be or include a multi-modal model such as GPT-4o which is configured to extract text from an input image (see, e.g., https://openai.com/index/hello-gpt-4o/).

The language model LLM is configured to recognize personal data in the text data. The language model LLM may be configured to consider additional data FD 1 for the recognition of personal data in texts.

The language model LLM can be configured and/or prompted to complete, clean and/or structure the text data in a meaningful way (e.g. respecting standard medical terms) before analysis. This "auto-corrective" processing step helps to improve the results provided by the text recognition module.

The language model LLM may be configured to recognize and specify different types of personal data in the text data.

The language model LLM may be configured to highlight personal data and/or personal data of a certain type, e.g. by highlighting it in a certain colour.

The language model LLM may be configured to provide, for personal data and/or types of personal data, an explanation of why it is personal data and/or personal data of a particular type.

The language model LLM may be configured to provide, for recognized personal data and/or for recognized personal data of a particular type, a probability that it is personal data and/or personal data of the particular type.

The language model LLM may be configured to provide, for recognized personal data a reasoning why the recognized data at hand contains PII/PHI. For example, the LLM can be prompted to take the HIPAA guidelines for PHI into account (see, e.g., https://www.hhs.gov/hipaa/for-professionals/privacy/laws-regulations/index.html).

The language model LLM may be configured to provide recognized personal data and/or the aforementioned information related to recognized personal data to the aggregation module AM, the decision module DM, and/or the personal data removal module PDRM.

The language model can be or include a LLAMA model (https://llama.meta.com) and/or a GPT model (https://openai.com/chatgpt/), for example.

It is possible for the language model LLM to be prompted by a text prompt to identify personal data and/or personal data of a certain type in the text data. It is possible that the computer system/computer program of the present disclosure provides a user interface via which the user can change and/or add to the text prompt. The text prompt can be displayed on a monitor and/or output via a loudspeaker, for example. Input from a user can be made, for example, by means of a keyboard, mouse and/or microphone.

The aggregation module AM is configured to aggregate the data supplied by the text region recognition module TRRM, the text recognition module TRM and/or the language model LLM.

The aggregation module AM may be configured to link the information about which texts contain personal data with the information about where these texts occur in the medical image MI.

The decision module DM is configured to determine based on the data provided whether and/or which notification is issued and/or whether and/or which personal data is to be removed from the medical image MI.

The decision module DM may be configured to compare the probability that the medical image MI comprises personal data with a threshold value and, depending on the result of the comparison, cause the reporting module RM to (i) output a notification, (ii) not output a notification, (iii) output a notification of a particular type, and/or (iv) output a (particular) notification to a particular recipient.

The decision module DM may be configured to compare the probability that the medical image MI comprises personal data with a threshold value and, depending on the result of the comparison, cause the patient data removal module PDRM to (i) remove text comprising personal data from the medical image MI, and/or (ii) remove text comprising personal data of a certain type from the medical image MI.

The reporting module RM is configured to issue notifications.

The term "issue a notification" may mean that a notification is output, e.g. displayed on a monitor and/or output via a loudspeaker and/or printed out by means of a printer, and/or that a notification is stored in a data storage, and/or that a notification is transmitted to a separate computer system.

The personal data removal module PDRM is configured to remove text representing personal data from the medical image MI. The personal data removal module PDRM may be or include a GAN (https://www.tensorflow.org/tutorials/generative/pix2pix), for example.

In an embodiment of the present disclosure, the architecture is hosted in a secure environment and access to the architecture is restricted to registered users to protect any personal data.

In an embodiment of the present disclosure, patient data comprising a medical image may be sent to the architecture over an encrypted connection, wherein the architecture is configured to recognize and/or remove personal data in the medical image and return a notification about the personal data and/or a medical image without the personal data over an encrypted connection.

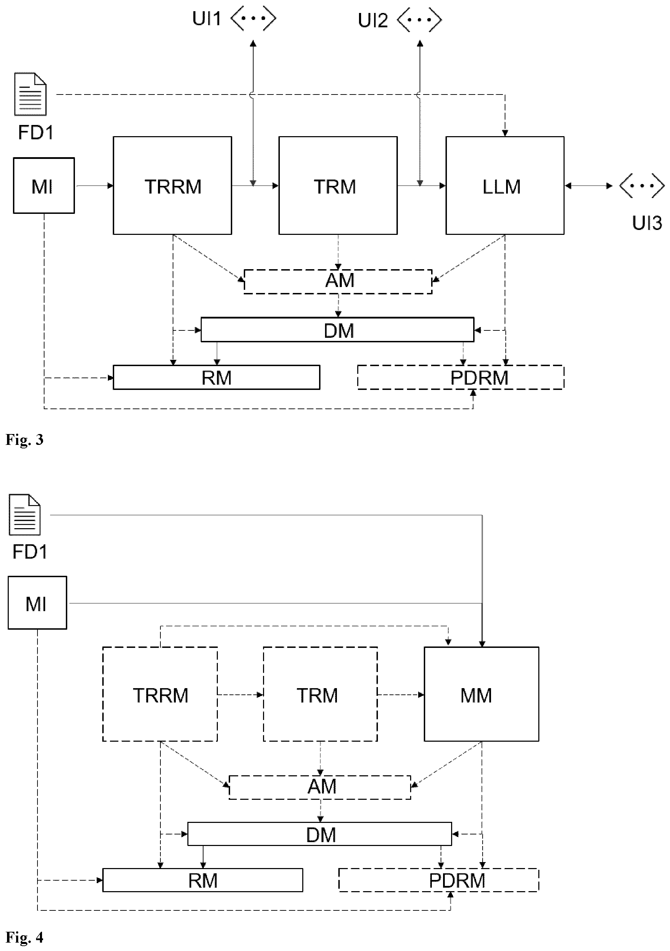

Fig. 3 shows a variant of the architecture shown in Fig. 2. In addition to the user interface UI3, with which a user can influence the text prompt for the language model LLM, there are two further ways in which a user can influence the computer-implemented procedure.

The user interface UI1 may be used to show the user the regions in which the text region recognition module TRRM has recognized text. This allows the user to check whether the text region recognition module TRRM has recognized all text regions. If the module has not recognized a region with text, it is possible for the user to mark this region (e.g. by drawing a virtual frame around the text with a computer mouse) so that this region is taken into account in the subsequent steps. It is also possible that the text region recognition module TRRM has recognized a region as a text region that does not contain any text at all. In such a case, the user can exclude this region from further analysis, e.g. by deleting a bounding box around the region.

The text data recognized by the text recognition module TRM can be displayed to a user via the user interface UI2. In addition to the text data, the text can also be displayed to the user in graphical form. The user can check whether all texts/characters have been recognized correctly. The user can make corrections to characters and/or words that have not been recognized correctly.

Actions performed by a user via user interfaces UI1, UI2 and/or UI3 can also be used to further train the modules/models of the architecture.

Fig. 4 shows a variant of the architectures shown in Fig. 2 and Fig. 3.

The language model has been replaced by a multimodal model MM. The multimodal model MM is configured to receive the medical image MI and the additional data FD1 and to recognize personal data in the medical image MI.

The text region recognition module TRRM and the text recognition module TRM are no longer needed as their functionalities are taken over by the multimodal model MM; however, they can still be used to provide the multimodal model MM with further data such as coordinates of text regions and/or graphical representations of text regions and/or text data for the recognition of personal data.

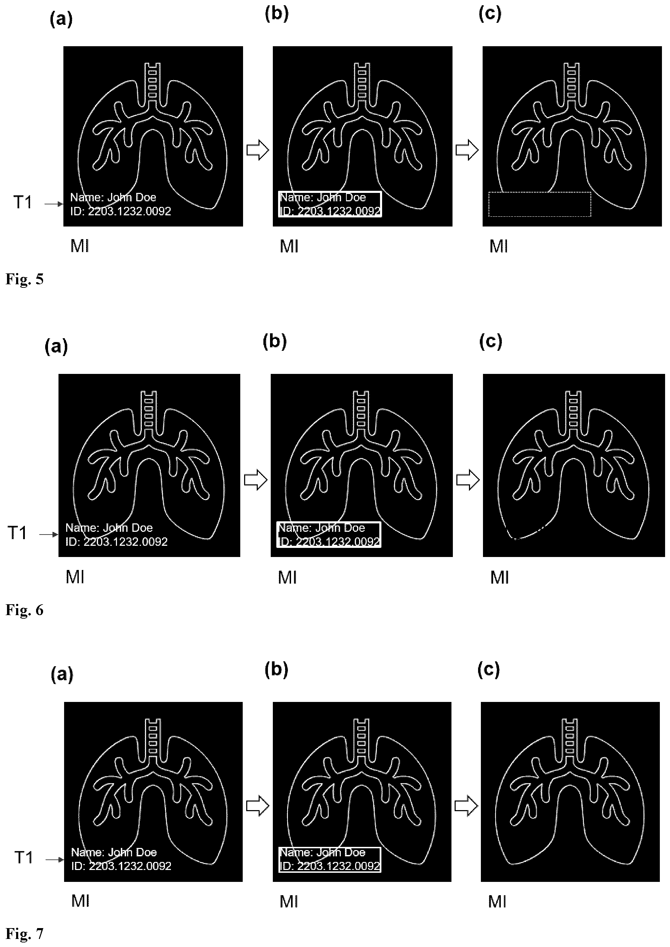

Fig. 5, Fig. 6 and Fig. 7 schematically show the removal of personal data in medical images.

Fig. 5(a) shows a medical image MI, which includes a text T1. The medical image MI shows a human lung.

Fig. 5(b) shows the result of a text recognition. The text T1 in the medical image MI was recognized; furthermore, it was recognized that the text T1 is personal data. The personal data was marked with a bounding box.

Fig. 5(c) shows one way of removing personal data. The colour values of the image elements within the text region representing personal data were set to zero (black). For better visibility, this region is marked with a dashed frame. As a result, parts of the medical image MI representing the human lung have also been set to zero. As a result, information is lost.

Fig. 6 shows another way of removing personal data. Fig. 6(a) and Fig. 6(b) correspond to Fig. 5(a) and Fig. 5(b).

In Fig. 6(c), those image elements that represent text have been set to zero (black). As a result, parts of the medical image MI representing the human lung have also been set to zero. As a result, information is lost.

Fig. 7 shows another way of removing personal data. Fig. 7(a) and Fig. 7(b) correspond to Fig. 5(a) and Fig. 5(b).

Fig. 7(c) shows the result of an image-to-image translation in which text was removed using an image-to-image translation model. In contrast to the methods shown in Fig. 5 and Fig. 6, no information is lost.

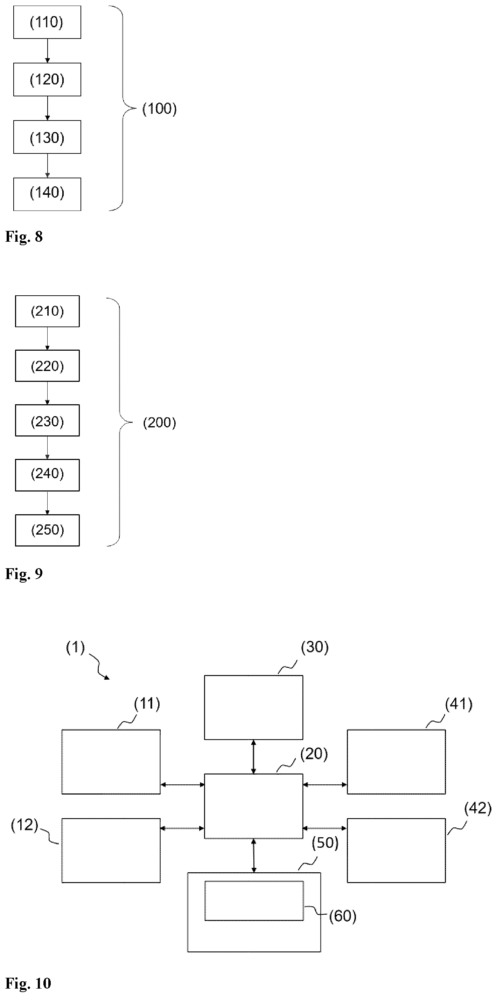

Fig. 8 shows an embodiment of the computer-implemented method of the present disclosure in the form of a flow chart.

The method (100) comprises the steps:
(110) receiving patient data, wherein the patient data comprises a medical image,
(120) recognizing text in the medical image,
(130) recognizing personal data in the text,
(140) issuing a notification about the personal data and/or deletion of the personal data.

Fig. 9 shows another embodiment of the computer-implemented method of the present disclosure in the form of a flow chart.

The method (200) comprises the steps:
(210) receiving patient data, wherein the patient data comprises a medical image,
(220) recognizing text in the medical image,
(230) recognizing personal data in the text,
(240) removing the personal data from the medical image,
(250) outputting the medical image without personal data, and/or storing the medical image without personal data in a data storage and/or transmitting the medical image without personal data to a separate computer system.

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

Fig. 10 illustrates a computer system (1) according to some example implementations of the present disclosure in more detail. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method comprising:
- receiving patient data, wherein the patient data comprises a medical image,
- recognizing text in the medical image,
- recognizing personal data in the text,
- issuing a notification about the personal data and/or deletion of the personal data.

2. The method of claim 1, wherein recognizing text in the medical image comprises one or more of the following:
- detecting one or more text regions in the medical image,
- extracting one or more text regions from the medical image,
- segmenting text regions in the medical image,
- segmenting text into lines, words, and/or individual characters,
- perspective correction and/or normalization,
- transcribing visual information of one or more text regions into text data,
- character recognition,
- spell checking and/or correcting the text and/or adding missing letters and/or words and/or characters.

3. The method of claim 1 or 2, wherein recognizing text in the medical image comprises correcting the text and/or adding missing letters and/or words and/or characters and/or complete, clean and/or structure the text using a pre-trained language model.

4. The method of any one of claims 1 to 3, wherein recognizing personal data in the text is carried out using a trained language model.

5. The method of claim 4, wherein the language model is configured to classify text into one of at least two classes, at least one class representing text that does not contain personal data and at least one class representing text that does contain personal data

6. The method of claim 4, wherein the language model is configured to classify text into one of at least three classes, one class representing text that does not contain personal data, one class representing text that contains personal data of a first type, and one class representing text that contains personal data of a second type.

7. The method of any one of claims 4 to 6, wherein the language model is configured to specify a probability for the correctness of the classification.

8. The method of any one of claims 4 to 6, wherein the language model is configured to receive the text and optionally further patient data and to indicate whether and/or to what extent the text contains personal data and/or contains personal data of a defined type.

9. The method of any one of claims 1 to 8, wherein the notification indicates whether the medical image contains personal data or not.

10. The method of any one of claims 1 to 9, wherein the notification indicates the probability that the medical image includes personal data.

11. The method of any one of claims 1 to 10, wherein the notification indicates what type(s) of personal data the medical image contains.

12. The method of any one of claims 1 to 11, wherein the notification includes the personal data.

13. The method of any one of claims 7 to 12, further comprising:
- comparing the probability that the medical image contains personal data with a defined threshold value and issuing the notification if the probability is greater than or equal to the threshold value.

14. The method of any one of claims 1 to 13, wherein the notification indicates where personal data is present in the medical image and/or where personal data is present with a probability above a threshold value or where personal data of a defined type is present in the medical image.

15. The method of any one of claims 1 to 14, wherein the notification includes the medical image in which text representing personal data is marked.

16. The method of any one of claims 7 to 15, wherein the notification includes an explanation of why the text is personal data and/or personal data of a particular type.

17. The method of any one of claims 7 to 16, wherein the notification includes a list of all texts identified in the medical image and an indication of whether the text is personal data, the probability that it is personal data, what type of personal data the text represents, the probability that the text represents the type of personal data and an explanation of why the text is personal data and/or personal data of a particular type.

18. The method of any one of claims 1 to 17, wherein an image-to-image translation model is used to delete text from the medical images.

19. The method of claims 18, wherein the image-to-image translation model was trained in a supervised learning task on training data comprising images with text and the corresponding images without text.

20. The method of claim 18 or 19, wherein the image-to-image translation model is or comprises a generative adversarial network or a diffusion model.

21. A computer system comprising:
a processing unit; and
a memory storing a computer program configured to perform, when executed by the processing unit, the method of any one of claims 1 to 20.

22. A non-transitory computer readable storage medium having stored thereon a computer program that, when executed by a processing unit of a computer system, cause the computer system to execute the method of any one of claims 1 to 20.
